# EUROPEAN PATENT APPLICATION

(11) **EP 4 700 130 A1**
(43) Date of publication of application: **25.02.2026**
(21) Application number: 23945541.3
(22) Date of filing: 16.08.2023
(51) Int. Cl.: C12P 21/02, C07K 5/062, C12N 9/10

(54) **PREPARATION METHOD FOR ALPHA-AMINO ACID ESTER ACYL TRANSFERASE AND USE THEREOF IN DIPEPTIDE SYNTHESIS**

(30) Priority: 20.07.2023 CN 202310894437
(71) Applicant: Asymchem Life Science (Tianjin) Co., Ltd, Tianjin 300457 (CN)
(72) Inventor: HONG, Hao, Morrisville, North Carolina 27560 (US); JAMES, Gage, Morrisville, North Carolina 27560 (US); ZHANG, Na, Tianjin 300457 (CN); JIAO, Xuecheng, Tianjin 300457 (CN); MA, Yulei, Tianjin 300457 (CN); ZHANG, Kejian, Tianjin 300457 (CN); SUN, Huanhuan, Tianjin 300457 (CN); LI, Fang, Tianjin 300457 (CN); LI, Chengyu, Tianjin 300457 (CN); AN, Xiangxiang, Tianjin 300457 (CN); SU, Lankai, Tianjin 300457 (CN); QI, Yike, Tianjin 300457 (CN); KANG, Yu, Tianjin 300457 (CN); LI, Hongxuan, Tianjin 300457 (CN); MA, Tianjiao, Tianjin 300457 (CN)
(74) Representative: Biggi, Cristina
(86) International application number: PCT/CN2023/113396
(87) International publication number: WO 2025/015656

(57) **Abstract**

Provided are a method for preparing an α-amino acid ester acyltransferase and a use thereof in dipeptide synthesis. The method for dipeptide synthesis includes: using α-amino acid ester acyltransferase to perform a dipeptide synthesis reaction, and obtaining a dipeptide; where the α-amino acid ester acyltransferase has a conserved region with the amino acid sequence as shown in SEQ ID NO: 1. Using the α-amino acid ester acyltransferase of the present application, a plurality of functional dipeptides can be synthesized, a substrate spectrum is wide, and the synthesis efficiency of some enzymes is high. It can be well used for industrial scale-up, with low cost and high yield, achieving true green chemistry.

## Description

The present application is based on and claims priority to Chinese Application No. 202310894437.3, filed on July 20, 2023, the invention of which is incorporated into the present application in its entirety.

### Technical Field

The present invention relates to the field of enzyme technology, and specifically, to a method for preparing an α-amino acid ester acyltransferase and a use thereof in dipeptide synthesis.

### Background

A dipeptide is the simplest peptide, consisting of an amide bond formed by dehydration condensation between the a-carboxyl group of one amino acid molecule and the α-amino group of another amino acid molecule. Although the structure of the dipeptide is simple, it has a wide range of biological activities and is able to regulate life activities such as physiological metabolism of organisms. The demand for peptides based on antibiotic, antiviral, anticancer, hormonal, and immunomodulatory effects has been increasing year by year, and these peptides are widely used in fields such as medicine, food, health products, and cosmetics. For example, carnosine (β-alanyl-His) has antioxidant, anti-inflammatory, and anti-glycation effects; glycyl-glycine (Gly-Gly) is used in medicine as a stabilizer for blood preservation and a protein drug cytochrome C aqueous injection; alanyl-glutamine (Ala-Gln) serves as an important nutritional supplement for surgical patients; aspartame (Asp-Phe methyl ester) is a widely used sweetener; Ala-Phe, Ile-Phe, and Pro-Gly dipeptides are saltiness enhancers; Ile-Tyr, Lys-Trp, Val-Tyr, and Ile-Trp are antihypertensive peptides; Arg-Trp has analgesic effects; and Lys-Glu exhibits antitumor activity, and so on. Although an increasing number of functional dipeptides have been discovered, only aspartame and Ala-Gln have achieved commercial production at present. The chemical synthesis of dipeptides has certain limitations, such as involving protection and deprotection operations of amino acids during the process, racemization of the product, high synthesis costs, and even the need to add toxic reagents. Therefore, finding a suitable and efficient method for dipeptide synthesis has always been a hot topic.

An α-amino acid ester acyltransferase (Aet) can utilize alanine methyl ester hydrochloride as an acyl donor to react with another nucleophile, glutamine, to form a Ala-Gin dipeptide, and the entire process does not require ATP participation and exhibits high synthesis efficiency (A novel and efficient enzymatic method for the production of peptides from unprotected starting materials. Biotechnol. 2005 Jan 26; 115(2):211-20.; Gene cloning and characterization of α-amino acid ester acyl transferase in Empedobacter brevis ATCC14234 and Sphingobacterium siyangensis AJ2458. Biosci Biotechnol Biochem. 2011; 75(11):2087-92.). Currently reported Aet exhibits high catalytic efficiency but generally has a narrow substrate spectrum, and only a small number of dipeptides (no more than 30 types) have been reported to be synthesizable using Aet, which limits the widespread application of Aet in synthesizing a plurality of dipeptide products. Therefore, it is of great significance to develop an α-amino acid ester acyltransferase with a broad substrate spectrum.

### Summary

The main objective of the present invention is to provide a method for preparing an α-amino acid ester acyltransferase and use thereof in dipeptide synthesis, so as to solve the problem of the narrow substrate spectrum of the α-amino acid ester acyltransferase in the prior art.

To achieve the above objective, according to an aspect of the present invention, provided is a method for preparing a dipeptide, which comprises: using an α-amino acid ester acyltransferase to perform a dipeptide synthesis reaction to obtain the dipeptide; where the α-amino acid ester acyltransferase has a conserved region with an amino acid sequence as shown in SEQ ID NO: 1.

Furthermore, the α-amino acid ester acyltransferase is selected from any one or more of the α-amino acid ester acyltransferases with amino acid sequences as shown in SEQ ID NOs: 2-11.

Furthermore, the substrates for the dipeptide synthesis reaction comprise a substrate as an acyl donor and a substrate as a nucleophile, wherein the substrate as an acyl donor is selected from an amino acid ester hydrochloride, and the substrate as a nucleophile is an amino acid; preferably, the amino acid ester hydrochloride comprises an amino acid methyl ester hydrochloride, an amino acid ethyl ester hydrochloride, or an amino acid isopropyl ester hydrochloride; preferably, the amino acid in the amino acid ester hydrochloride and the amino acid as a nucleophile are each independently selected from glycine, alanine, valine, leucine, isoleucine, serine, threonine, cysteine, methionine, asparagine, glutamine, aspartic acid, glutamic acid, lysine, arginine, histidine, phenylalanine, tryptophan, proline, or tyrosine.

Furthermore, the dipeptide comprises Gly-Gln, Gly-Tyr, Gly-Gly, Ala-Gln, or Arg-Trp.

Furthermore, the dipeptide synthesis reaction is performed in an alkaline environment.

Furthermore, the alkaline environment is an alkaline buffer, preferably the alkaline buffer comprises 0.1-0.2 M Tris-HCl with a pH of 8-8.5.

Furthermore, the reaction temperature for the dipeptide synthesis is 20-25°C; preferably, the reaction time is 1.5-2 h.

Furthermore, after the dipeptide synthesis reaction, the method further comprises heating and deactivating the α-amino acid ester acyltransferase. A temperature for the heating and deactivating is 75-85°C; preferably, a time for the heating and deactivating is 10-15 min.

Furthermore, based on the mass ratio of a wet bacterial sludge to the substrate, the mass ratio of the wet bacterial sludge comprising the α-amino acid ester acyltransferase to the substrate for the dipeptide synthesis reaction is 1:1-10, preferably 1:10.

According to another aspect of the present invention, provided is a method for preparing an α-amino acid ester acyltransferase, wherein the gene of the α-amino acid ester acyltransferase in the above method is cloned into an expression vector to obtain a recombinant vector; the recombinant vector is transformed into *Escherichia coli* to obtain a recombinant strain; the recombinant strain is cultured and induced to express the α-amino acid ester acyltransferase to obtain a culture solution; the culture solution is subjected to ultrasonic disruption and centrifugation to obtain the α-amino acid ester acyltransferase; preferably, the expression vector comprises pET-22a(+), pET-22b(+), pET-3a(+), pET-3d(+), pET-11a(+), pET-12a(+), pET-14b(+), pET-15b(+), pET-16b(+), pET-17b(+), pET-19b(+), pET-20b(+), pET-21a(+), pET-23a(+), pET-23b(+), pET-24a(+), pET-25b(+), pET-26b(+), pET-27b(+), pET-28a(+), pET-29a(+), pET-30a(+), pET-31b(+), pET-32a(+), pET-35b(+), pET-38b(+), pET-39b(+), pET-40b(+), pET-41a(+), pET-41b(+), pET-42a(+), pET-43a(+), pET-43b(+), pET-44a(+), pET-49b(+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-18, pUC-18, or pUC-19; preferably, host cells comprise prokaryotic cells or eukaryotic cells; preferably, the prokaryotic cells are *Escherichia coli* or *Bacillus subtilis*; more preferably, the strain of *Escherichia coli* is BL21; preferably, the eukaryotic cells are *Saccharomyces cerevisiae* or *Pichia pastoris*; preferably, the 3' end or 5' end of the gene of the α-amino acid ester acyltransferase in the recombinant vector comprises a tag gene; preferably, the tag gene comprises a DNA sequence encoding an MBP protein, a NusA protein, a Trx protein, a SUMO protein, a DsbA protein, a TF protein, or a GST protein; more preferably, the tag gene comprises a DNA sequence encoding an MBP protein, a SUMO protein, or a TF protein.

By applying the technical solution of the present invention and using the α-amino acid ester acyltransferase of the present application, a plurality of functional dipeptides can be synthesized, a substrate spectrum is wide, and the synthesis efficiency of some enzymes is high. It can be well used for industrial scale-up, with low cost and high yield, achieving true green chemistry.

### Detailed Description of the Embodiments

It should be noted that the embodiments and the features of the embodiments in the present application can be combined with each other under the circumstances that there is no conflict. The present invention will be described in detail below with reference to embodiments.

As mentioned in the background, the α-amino acid ester acyltransferase in the prior art generally has a narrow substrate spectrum in dipeptide synthesis reactions, and only a small number of dipeptides (no more than 30 types) have been reported to be synthesized using the α-amino acid ester acyltransferase, which limits the widespread application of the α-amino acid ester acyltransferase in synthesizing a plurality of dipeptide products. Therefore, it is of great significance to develop an α-amino acid ester acyltransferase with a broad substrate spectrum. In the present application, the inventors have obtained, through large-scale screening, a series of α-amino acid ester acyltransferases that can be widely used for producing functional dipeptides, wherein the screened enzymes can catalyze the synthesis of more than 200 types of dipeptides, significantly broadening the market for dipeptide product production, and thus have proposed a series of protection schemes in the present application.

In a first typical embodiment of the present invention, provided is a method for preparing a dipeptide, which comprises: using an α-amino acid ester acyltransferase to perform a dipeptide synthesis reaction to obtain the dipeptide; where the α-amino acid ester acyltransferase has a conserved region with an amino acid sequence as shown in SEQ ID NO: 1.
SEQ ID NO: 1:
YGISYPGFYST.

Through sequence alignment of amino acid sequences of a series of α-amino acid ester acyltransferases, the present application identifies a class of enzymes with the aforementioned conserved amino acid sequence regions, which are crucial for synthesizing a wide variety of dipeptides. In a preferred embodiment, the above α-amino acid ester acyltransferase is selected from any one or more of the α-amino acid ester acyltransferases with amino acid sequences as shown in SEQ ID NOs: 2-11. The aforementioned α-amino acid ester acyltransferase has an amino acid sequence of a wild-type α-amino acid ester acyltransferase derived from species *Sphingobacterium* and *Pedobacter.* It should be noted that the present application is not limited to these enzymes, and any wild-type or modified α-amino acid acyltransferase comprising the aforementioned conserved amino acid sequence regions is applicable to the present application.

Families of amino acid residues with similar side chains have been defined in the art. These families comprise amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). Therefore, it is preferable to replace the corresponding amino acid residue with another amino acid residue from the same side chain family. Methods for identifying conservative amino acid substitutions are well known in the art (see, e.g., Brummell et al., Probing the combining site of an anti-carbohydrate antibody by saturation-mutagenesis: role of the heavy-chain CDR3 residues. Biochem. 32:1180-1187 (1993); Kobayashi et al., Tryptophan H33 plays an important role in pyrimidine (6-4) pyrimidone photoproduct binding by a high-affinity antibody. Protein Eng. 12(10):879-884 (1999); and Burks et al., In vitro scanning saturation mutagenesis of an antibody binding pocket. Proc Natl Acad Sci USA. 94:412-417 (1997)).
SEQ ID NO: 2: 
MKKGITIFSALLIAFSFKAAAQSTAESNYVKEHYEKTEIAIPMRDGVKLFTTIYSPKDKSQSYPVL
LNRTPYTVGPYGPNEYKPSLGNFPAMAKEGYIFVYQDVRGKWMSEGTFEDVRPTTLKKGKKDIDE
STDTYDLLEWLSKNLKNYNGKAGMYGISYPGFYSTVSLVKTHPSLKAVSPQAPVTNWYIGDDFHH
NGVLFTGDAFKFMSSFGIPRPQPITPNQAPHTFQYPSKDVYQFYLTGGTAKDLKNTYFADSVKFW
NDAFAHPHYDQFWKDRLILPHLTNVKPAVMIVGGFFDAEDAYGTFETYKKIEEQNPQNKSILVAGP
WFHGGWVRSDGSSFGDIQFDQKTSVTYQEKFELPFFNYYLKGKGDFKAAEANIFVTGSNQWKSF
DKWPPKDVEDKKLYLQPQGKLDFEKVGRTDSWDEYVSDPNKPVPYQGGVMESRTREYMIDDQR
FASSRPDVMVYQTEPLTADITIVGPIKNFLKVSTSGTDADYVVKLIDVYPANSPSFNNKVMDGYQML
VRGEIMAGKYRNSFEKAEPMQPGFVTDIKYTMPDVAHTFKKGHRIMIQVQNTWFPIAERNPQQFF
EGYEATATDYRKATHRIFHDVNNASYIEFSVLK.
SEQ ID NO: 3: 
MKNTISYLTLALLSASQLHAQTAADSAYVRDHYEKTEVAIPMRDGKKLFTAIYSPKDKSKKYPV
LLNRTPYTVSPYGQNEYKKSLGNFPQMMHEGYIFVYQDVRGKWMSEGDFEDIRPTTYSKDKKAID
ESTDTYDALEWLQKNLKNYNGKAGLYGISYPGFYSTVGLVKTHPSLKAVSPQAPVTDWYIGDDFH
HNGVLFLQDAFTFMSTFGVPRPKPITPDQFKGKIQIKEADKYNFFAEAGTARELKEKYFGDSVQFW
NDLFKHPDYDDFWKSRVITNSLQEVRPAVMVVGGFFDAEDAYGTFKTYQSIEDKSKKNNSILVAGP
WYHGGWVRAEGNYLGDIQFEKKTSITYQEQFEQPFFKYYLKDEGNFAPSEANIFVSGSNEWKHF
 
EQWPPKNVETKKLYFQPQGKLGFDKVQRTDSWDEYVTDPNKPVPHQGGLIQNRTREYMVDDQR
FAASRPDVMVYQTEPLTEDLTIVGPIKNFLKVSSTGTDADYVVKLIDVYPNDAASYQGKTMAGYQM
MVRGEIMAGKYRNGFDKAQALTPGMVEKVNFEMPDVAHTFKKGHRIMVQVQNSWFPLAERNPQ
VFLAPYTATKADFRKATQRIFHDVNNATYIEFSVLKD.
SEQ ID NO: 4: 
MKNTISCLTLALLSASQLHAQTAADSAYVRDHYEKTEVAIPMRDGKKLFTAIYSPKDKSKKYPV
LLNRTPYTVSPYGQNEYKKSLGNFPQMMREGYIFVYQDVRGKWMSEGDFEDIRPTTYNKDKKAI
DESTDTYDALEWLQKNLKNYNGKAGLYGISYPGFYSTVGLVKTHPSLKAVSPQAPVTDWYIGDDF
HHNGVLFLQDAFTFMSTFGVPRPKPITPDQFKGKIQIKEADKYNFFAEAGTARELKEKYFGDSVQF
WNDLFKHPDYDDFWKSRVITNSLQEVKPAVMVVGGFFDAEDAYGTFKTYQSIEDKSKRNNSILVA
GPWYHGGWVRAEGNYLGDIQFEKKTSITYQEQFEQPFFKYYLKDEGNFAPAEANIFVSGSNEWK
HFEQWPPKNVETKKLYFQPQGKLGFDKVQRTDSWDEYVTDPNKPVPHQGGLIQNRTREYMVDD
QRFAASRPDVMVYQTEPLTEDLTIVGPIKNFLKVSSTGTDADYVVKLIDVYPNDAASYQGKTMAGY
QMMVRGEIMAGKYRNGFDKAQALTPGMVEKVNFEMPDVAHTFKKGHRIMVQVQNSWFPLAERN
PQVFLPSYTATKADFRKATQRIFHDVNNATYIEFSVLKD.
SEQ ID NO: 5: 
MKNTISYLTLALLSASQLHAQTPADSAYVRDHYEKTEVAIPMRDGKKLFTAIYSPKDKSKKYPV
LLNRTPYTVSPYGQNEYKKSLGNFPAMMREGYIFVYQDVRGKWMSEGNFEDIRPTTYSKDKKAF
DESTDTYDALEWLQKNLKNYNGKAGLYGISYPGFYSTVGLVKTHPSLKAVSPQAPVTDWYIGDDF
HHNGVLFLQDAFTFMSTFGVPRPKPITPDQFKSKIQIKEADKYNFFLEAGTARELKEKYFGDSVQF
WNDLFKHPDYDDFWKSRVITNSLQEVKPAVMVVGGFFDAEDAYGTFKTYQSIEDKSKKNNSILVAG
PWYHGGWVRAEGNYLGDIQFEKKTSVTYQEQFEQPFFKYYLKDEGNFAPSEANIFVSGSNEWKH
FEQWPPKNVEIKKLYFQPQGKLGFDKVQRTDSWDEYVTDPNKPVPHQGGLIQNRTREYMVDDQ
RFAASRPDVMVYQTEPLTEDLTIVGPIKNFLKVSSTGTDADYVVKLIDVYPNDAASYQGKTMAGYQ
MMVRGEIMAGKYRNGFEKAQALTPGMVEKVNFEMPDVAHTFKKGHRIMVQVQNSWFPLAERNP
QVFLAPYTATKADFRKATQRIFHDVNNATYIEFSVLKD.
SEQ ID NO: 6: 
MPRIKHLLFILLFSYGLSASAQNIDSIYVRENYTKIERSIPMRDGVKLFTAIYIPKDQNRKYPFLIN
RTPYTVAPYGADKYKLSLGNFPAMMREGFIFVYQDVRGKWMSEGTFDDIRPHIANKKSKKQIDES
SDTYDTIDWLVKNIKNNNGKAGIYGISYPGFYSTASLPESHPALKAVSPQAPVTDWFIGDDFHHNG
VLFLMDAFSFMTTFGVPRPKPITPDLGPKGLKFPIQDNYRFYLEAGSIKNLKDKYMGDSIRFWNNLF
AHPNLDTFWQARNIQRHLKSVKPAVMVVGGFFDAEDAYGTFSTYKSIEKQNPSANNILVMGPWYH
 
GGWVRSTGSSFGDIEFGQPTSLQYQERFELPFFKYYLKGQGDFKPAEANIFVTGSNEWKSFGTW
PPKDTEQKTLYLQPNGKLSFDKVQRTDSWDEYVSDPNTPVPYQEGVQAKRTREYMIDDQRFAAR
RPDVKVYQTDALTEDITLTGPVLANLVVSTTGTDADYVVKLIDVYPEDAPAPVPNPKNILMGGFQML
VRGEIMRGKYRNSFEKPEAMVPGLVTKVNYALPDVAHTFRKGHKIMIQVQNSWFPLGDRNPQKF
MDIYTAEPSDFQKATQRIFHDVNNASSITISVLK.
SEQ ID NO: 7: 
MNLTRIFSLLFCLLIANQLMAQQTDSAYVRENYTKIERQIPMRDGVKLFTSIYIPKNQSKKYPFLI
NRTPYTVAPYGEDKYKLSLGNFPAMMREGFIFVYQDVRGRWMSEGTFADIRPQQTGKKSKNAIDE
STDTYDTIDWLVKNVKGNNGNAGIYGISYPGFYSTTSLPNAHPALKAVSPQAPVTDWFMGDDFHH
RGTLFLMDAFSFMGSFGVPRPKPITPDKGPKGFQFPIEDNYRFYLEAGSVKSLKDKYFADSIKFWN
DLFKHPNLDTFWKARLITPHLTNVKPAVMVVGGFFDAEDAYGTFATYKAIEKQNPGANNILVAGPW
FHGGWVRSDGSSFGDIPFGKTTSIDYQEQYELPFFKHYLKGEGDFNAAEANIFVTGSNEWKKFKT
WPPQGVESKNLYLQPNGKLAFEKVGRTDSWDEYVSDPNNPVPYQDGIQSKRTREYMIDDQRFAA
RRPDVKSYQTDALTEDITLTGPVLANLVVSTTGTDADYVVKLIDVYPEDAPNPEPNPKNLIMGGYE
MLVRGEIMRGKYRNSFEKPEAFIPGTITKVNYPLPDVAHTFKKGHKIMIQIQNSWFPLADRNPQKFM
DIYQAEPQDFQKATHKIYHDVHNSSYITVSVLK.
SEQ ID NO: 8: 
MKKVLIIFSVFLITISLQVKAQVTLEPNYVKEHYEKKEIAIPMRDGVKLFTTIYTPKDKSQQYPVL
LNRTPYTVGPYGEDKFKESLGNFPAMTTEGYIFVYQDVRGKWMSEGTFEDIRPTNLKKGKREIDE
STDTYDLLEWLSKNLQNYNGKAGMYGISYPGFYSTVSLVKTHPSLKAVSPQAPVTNWYMGDDFH
HNGILFTGDAFKFMSSFGIPRPKPITPDQAPQTFQYPSKDVYQFYLDAGTAKDLKDKYFADSVKFW
NDVFAHPHYDQFWKDREILPHVKNVKPAVMIVGGFFDAEDTYGTFETYKTIEKQNPNNNSILVVGP
WFHGGWVRGDGSSFGDIQFDQKTSVTYQEQFELPFFNYYLKGKGDFKASEANIFITGSNQWKAF
EKWPPQEVEDKKLYLQPQGKLGFDQVKRTDSWDEYVSDPNKPVPYQAGIMESRTREYMIDDQRF
ASSRPDVMVYQTEPLAADLTIVGPIKNFLKVSTSGTDADYVVKLIDVYPANSPSFNNKVMDGYQML
VRGEIMAGKYRNGFEKAEAMQPGFVTAIDYTMPDVAHTFKKGHRIMLQVQNTWFPLAERNPQQF
FEGYEATAGDYRKATHRIFHDVNNSSYIQFAILK.
SEQ ID NO: 9: 
MNLSKILGIVICLFVSHEMFAQQPDSAYVRENYTKIERQIPMRDGVKLFTSIYIPKNQSKKYPFLI
NRTPYTVAPYGEDKYKLSLGNFPAMMREGFIFVYQDVRGRWMSEGTFEDIRPQTLKKGKKDIDES
TDTYDTIDWLVKNIKNNNGNAGIYGISYPGFYSTTSLPNAHPALKAVSPQAPVTDWFIGDDFHHGG
ALFLMDAFSFMSGFGVPRPKPITPDKGPKGFNFPIQDNYRFYLESGSVKNLKDKYFADSIKFWNNL
 
VKHPNLDTFWKARYIIPHLKNVKPAVMVVGGFFDAEDAYGTFATYKAIEKQNPEANNILVAGPWFH
GGWVRSDGASFGDIKFGKTTSIDYQQQFELPFFKHYLKGEGDFKAAEANIFVTGSNEWKKFGSW
PPKEVETKNLYLQPNGKLAFEKVGRTDSWDEYVSDPNHPVPYQDGVQAKRTREYMIDDQRFAAR
RPDVKTYQTDALKDDITLTGPVLANLVVSTTGTDADYVVKLIDVYPEDEPNPSPNPNNIMMGGYEM
LVRGEILRGKYRNTFEKPEAFVPGAITKVNYALPDVAHTFKKGHKIMIQIQNSWFPLADRNPQKFMD
IYQAEPGDFQKATHKIYHDTSNSSYISVSVLK.
SEQ ID NO: 10: 
MKNTISCLTLALLSASQLHAQTAADSAYVRDHYEKTEVAIPMRDGKKLFTAIYSPKDKSKKYPV
LLNRTPYTVSPYGQNEYKKSLGNFPQMMREGYIFVYQDVRGKWMSEGDFEDIRPTTYSKDKKAID
ESTDTYDALEWLLKNLKNYNGKAGLYGISYPGFYSTVGLVKTHPSLKAVSPQAPVTDWFIGDDFHH
NGVLFLQDAFTFMSTFGVPRPKPITPDQFKGKIQIKEADKYNFFAEAGTARELKEKYFGDSVQFWN
DLFKHPDYDDFWKSRVITNSLQEVKPAVMVVGGFFDAEDAYGTFKTYQSIEDKSKKNNSILVAGPW
YHGGWVRAEGNYLGDIQFEKKTSITYQEQFEQPFFKYYLKDEGNFAPSEANIFVSGSNEWKHFEQ
WPPKNVETKKLYFQPQGKLGFDKVQRTDSWDEYVTDPNKPVPHQGGLIQNRTREYMVDDQRFA
ASRPDVMVYQTEPLTEDLTIVGPIKNFLKVSSTGTDADYVVKLIDVYPNDAASYQGKTMAGYQMMV
RGEIMAGKYRNGFDKAQALTPGMVEKVNFEMPDVAHTFKKGHRIMVQIQNSWFPLAERNPQVFL
APYTATKADFRKATQRIFHDVNNATYIEFSVLKD.
SEQ ID NO: 11:

MKTTISFLALTLFAGGQLHAQTNTDSAYVRDHYEKTEVTVPMRDGKKLFTVIYSPKDKSKKYPI
LLNRTPYTVGPYGQNEYKKSLGNFPAMMRDGYIFVYQDVRGKWMSEGDFEDVRPTNLSKDKKAI
DESTDTYDVLEWLQKNLQNYNGKAGLYGISYPGFYSTVGLVKTHPSLKAVSPQAPVTDWFIGDDF
HHNGVLFLQDAFTFMSTFGVPRPKPIRPNQFTSKIQINEVDKYNFFLEAGTARELKEKYFGDSVQF
WNNLFKHPNYDDFWKSRVITNYLQDVKPAVMVVGGFFDAEDAYGTFKTYQSIEEKSKTNNSVLVA
GPWYHGGWVRAAGDYLGDIQFEKKTSITYQEKFEQPFFSHYLKEEGSFSPAEANIFISGSNEWKQ
FEQWPPKNVETKKLYFQPQGKLGFEKVQRTDSWDEYVTDPNKPVPHQGGLIQNRTREYMVDDQ
RFAASRPDVMVYETAPLTEDITIVGPIKNFLKVASTGTDADYIVKLIDVYPSDAPSFDGKPMAGYQM
MVRGEIMAGKYRNGFDKPQPLTPGKVEKVDFVMPDVAHTFKKGHRIMVQVQNSWFPLAERNPQV
FMAPYTATKTDFRKATQRIFHDVNNATYIEFDILKD.

The α-amino acid ester acyltransferase (Aet) of the present application can synthesize dipeptides using a plurality of substrates, wherein an amino acid ester hydrochloride is used as an acyl donor to react with another amino acid as a nucleophile to generate a dipeptide. Therefore, any substrate that can be acted on by an α-amino acid ester acyltransferase is suitable for the present application. In a preferred embodiment, the substrate for the dipeptide synthesis reaction comprises a substrate as an acyl donor and a substrate as a nucleophile, wherein the substrate as the acyl donor is selected from an amino acid ester hydrochloride and the substrate as the nucleophile is an amino acid. In a preferred embodiment, the amino acid ester hydrochloride comprises an amino acid methyl ester hydrochloride, an amino acid ethyl ester hydrochloride, or an amino acid isopropyl ester hydrochloride, wherein using these three amino acid ester hydrochlorides as substrates enables highly efficient synthesis of dipeptides. More preferably, the amino acid ester hydrochloride comprises the amino acid methyl ester hydrochloride.

The types of amino acids in any substrate capable of utilizing the α-amino acid ester acyltransferase (Aet) of the present application for dipeptide synthesis are applicable to the present application. In a preferred embodiment, the amino acid in the amino acid ester hydrochloride and the amino acid as a nucleophile are each independently selected from glycine, alanine, valine, leucine, isoleucine, serine, threonine, cysteine, methionine, asparagine, glutamine, aspartic acid, glutamic acid, lysine, arginine, histidine, phenylalanine, tryptophan, proline, or tyrosine.

Among them, the reaction equation for dipeptide synthesis is as follows:

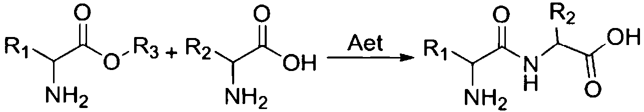

R₁ and R₂ represent side chains of 20 amino acids; and R₃ represents methyl, ethyl or isopropyl.

The α-amino acid ester acyltransferase in the present application has a broader range of substrates and can synthesize a greater variety of dipeptides; thus, in practical applications, any required dipeptide can be synthesized according to actual conditions. In a preferred embodiment, the synthesized dipeptide comprises Gly-Gln, Gly-Tyr, Gly-Gly, Ala-Gln or Arg-Trp. Particularly some important functional dipeptides are synthesized using α-amino acid ester acyltransferase. Among them, the dipeptide consists of an amide bond formed by dehydration condensation between the α-carboxyl group of one amino acid molecule and the α-amino group of another amino acid molecule, and is widely used in fields such as medicine, food, health products, and cosmetics. For example, glycyl-glutamine (Gly-Gln) can replace glutamine as a nutrient for cell culture to increase cell yield; glycyl-tyrosine (Gly-Tyr) is an antihypertensive peptide; glycyl-glycine (Gly-Gly) is used in medicine as a stabilizer for blood preservation and a protein drug cytochrome C aqueous injection; alanyl-glutamine (Ala-Gln) is used as an important nutritional supplement for surgical patients; and Arg-Trp has analgesic effects.

In a preferred embodiment, the dipeptide synthesis reaction of the present application is performed in an alkaline environment. Any condition capable of maintaining an alkaline environment for the dipeptide synthesis reaction using α-amino acid ester acyltransferase is applicable to the present application. In a preferred embodiment, the alkaline condition is an alkaline buffer. In a preferred embodiment, the alkaline buffer comprises 0.1-0.2 M Tris-HCI with a pH of 8-8.5.

Any reaction conditions capable of completing the dipeptide synthesis reaction are applicable to the present application. From the perspective of reaction efficiency and energy consumption of the dipeptide, in a preferred embodiment, the reaction temperature for the dipeptide synthesis reaction is 20-25°C; and the reaction time is 1.5-2 h. Under the reaction temperature and time conditions, the reaction is highly efficient and consumes relatively less energy.

In a preferred embodiment, after the dipeptide synthesis reaction is completed, the method further comprises heating and deactivating the α-amino acid ester acyltransferase. After the reaction, the α-amino acid ester acyltransferase is inactivated, wherein the enzyme is inactivated in time to stop the synthesis reaction, which is beneficial to improve the reaction efficiency of dipeptide synthesis and avoiding the impact of prolonged reaction time on the quality of dipeptide products. In a preferred embodiment, the dipeptide synthesis reaction is followed by heating and deactivating and the temperature for the heating and deactivating is 75-85°C; and the time for the heating and deactivating is 10-15 min.

Any ratio of enzyme and substrate utilizing α-amino acid ester acyltransferase for dipeptide synthesis reactions is applicable to the present application. To further improve the reaction efficiency of dipeptide synthesis, in a preferred embodiment, based on the mass ratio of wet bacterial sludge to substrate, the mass ratio of the wet bacterial sludge comprising the α-amino acid ester acyltransferase to the substrate for the dipeptide synthesis reaction is 1:1-10, preferably 1:10.

In a second typical embodiment of the present invention, a method for preparing an α-amino acid ester acyltransferase is provided, wherein the gene of the above α-amino acid ester acyltransferase is cloned into an expression vector to obtain a recombinant vector; the recombinant vector is transformed into *Escherichia coli* to obtain a recombinant strain; the recombinant strain is cultured and induced to express the α-amino acid ester acyltransferase, thereby obtaining a culture solution; and the culture solution is subjected to ultrasonic disruption followed by centrifugation to obtain the α-amino acid ester acyltransferase. The expression and production of the α-amino acid ester acyltransferase are accomplished using conventional prokaryotic protein expression methods.

Unless specifically indicated, the molecular biology experimental methods used in the present application are substantially performed according to the methods described in J. Sam brook et al., Molecular Cloning: A Laboratory Manual, 2nd Edition, Cold Spring Harbor Laboratory Press, 1989, and F. M. Ausubel et al., Short Protocols in Molecular Biology, 3rd Edition, John Wiley & Sons, Inc., 1995; and the use of restriction endonucleases follows the conditions recommended by the product manufacturers. Those skilled in the art understand that the embodiments describe the present application by way of example and are not intended to limit the scope claimed by the present application.

Any recombinant plasmid capable of normally expressing α-amino acid ester acyltransferase is applicable to the present application. In a preferred embodiment, the expression vector comprises pET-22a(+), pET-22b(+), pET-3a(+), pET-3d(+), pET-11a(+), pET-12a(+), pET-14b(+), pET-15b(+), pET-16b(+), pET-17b(+), pET-19b(+), pET-20b(+), pET-21a(+), pET-23a(+), pET-23b(+), pET-24a(+), pET-25b(+), pET-26b(+), pET-27b(+), pET-28a(+), pET-29a(+), pET-30a(+), pET-31b(+), pET-32a(+), pET-35b(+), pET-38b(+), pET-39b(+), pET-40b(+), pET-41a(+), pET-41b(+), pET-42a(+), pET-43a(+), pET-43b(+), pET-44a(+), pET-49b(+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-18, pUC-18, or pUC-19. Any host cell capable of normally inducing the expression of the α-amino acid ester acyltransferase is applicable to the present application. In a preferred embodiment, the host cells comprise prokaryotic cells or eukaryotic cells; the prokaryotic cells are *Escherichia coli* or *Bacillus subtilis*; the strain of *Escherichia coli* is BL21; and the eukaryotic cells are *Saccharomyces cerevisiae* or *Pichia pastoris.* Any gene element or sequence capable of promoting the expression of the gene of the α-amino acid ester acyltransferase gene is applicable to the present application. In a preferred embodiment, the 3' end or 5' end of the gene of the α-amino acid ester acyltransferase in the recombinant vector comprises a tag gene. In a preferred embodiment, the tag gene comprises a DNA sequence encoding an MBP protein, a NusA protein, a Trx protein, a SUMO protein, a DsbA protein, a TF protein, or a GST protein; and in a more preferred embodiment, the tag gene comprises a DNA sequence encoding an MBP protein, a SUMO protein, or a TF protein. The recombinant vector with the aforementioned tag gene is able to promote the expression of the gene of the α-amino acid ester acyltransferase and produce the α-amino acid ester acyltransferase more efficiently.

The following provides a further detailed description of the present application in conjunction with specific embodiments, which should not be construed as limiting the scope of protection claimed by the present application.

**Example 1 Preparation of α-Amino Acid Ester Acyltransferase Aet Enzyme Solution**

The enzyme sources of the enzyme library were obtained through literature retrieval and gene mining, then α-amino acid ester acyltransferase (Aet) from different sources was obtained by artificial chemical synthesis method, an endonuclease site Ndel was introduced at the 5' end and an endonuclease site Xhol was introduced at the 3' end, and the gene was synthesized into pUC19 to obtain pUC19-Aet. The expression vector pET-28a(+) was double-digested (Ndel+Xhol), and the gene pUC19-Aet of the α-amino acid ester acyltransferase was simultaneously double-digested (Ndel+Xhol). The gene fragment encoding Aet was excised and ligated to the expression vector pET-28a(+) to obtain the recombinant plasmid pET-28a(+)-Aet of *Escherichia coli* comprising the α-amino acid ester acyltransferase, which was then transformed into *Escherichia coli* BL21(DE3) to obtain a recombinant *Escherichia coli* strain BL21(DE3)/Aet. Additionally, to improve protein expression, different fusion tags such as MBP (maltose-binding protein), NusA (transcription termination anti-termination factor), Trx (thioredoxin A), SUMO (small ubiquitin-related modifier protein), DsbA (protein disulfide bond folding isomer), TF (trigger factor), and GST (glutathione S-transferase) were constructed at the N-terminus of the protein via homologous recombination for fusion expression. The protein expression levels with the added fusion tags were all improved to varying degrees.

4 mL of the BL21(DE3) strain containing the recombinant plasmid was taken and inoculated into a 2 L Erlenmeyer flask containing 400 mL of LB medium, and cultured with shaking at 37°C and 200 rpm for 2-3 h. When the OD₆₀₀ reached 0.6-0.8, IPTG was added to a final concentration of 0.02 mM, induced at 16°C for 18 h, and after induction, centrifuged at 4°C to collect bacterial cells. 10 mL of 0.1 M Tris-HCI (pH 8.0) was used to resuspend the bacterial cells per g of sludge, and subjected to ultrasonic disruption followed by centrifugation, then the supernatant was collected to obtain a crude enzyme solution for the catalytic reaction.

Note: The experimental materials and reagents used in the embodiments were described as follows:
1. Strains and vectors: *Escherichia coli* expression vector pET-28a(+) and strain BL21(DES3)
2. Medium: *E. coli* medium LB (10 g/L peptone, 5 g/L yeast extract, 10 g/L NaCl, pH 7.0).

### Example 2 Enzyme Screening

In this embodiment, more than 200 α-amino acid ester acyltransferases from the enzyme library and unreported enzymes that might have the same function were selected for screening, all of which were screened using the synthetic dipeptide Gly-Tyr. The screening system (1 mL) comprised: 12.5 mg (100 mM) glycine methyl ester hydrochloride, 18.1 mg (100 mM) tyrosine, 62.5 µL of crude enzyme solution, 0.1 M Tris-HCI (pH 8.0), and the reaction was carried out at 20°C for 2 h. The reaction system was heated at 80°C for 10 min, centrifuged, and then subjected to HPLC analysis. The reaction results of the enzymes with activity were statistically analyzed as follows (Table 1). Among them, 190 enzymes other than the 10 enzymes listed in the table showed no reaction activity, with a molar conversion rate of 0%.

**Table 1:**

| SEQ ID NO: | Sequence source | NCBI sequence number | Conversion rate |
|---|---|---|---|
| 2 | Sphingobacterium sp. SRCM116780 | WP_234514695.1 | ++ |
| 3 | Sphingobacterium paramultivorum | WP_182332756.1 | + |
| 4 | Sphingobacterium siyangense | WP_120334743.1 | + |
| 5 | Sphingobacterium sp. Ag1 | WP_046675788.1 | ++ |
| 6 | Pedobacter sp. | RZK77448.1 | + |
| 7 | Pedobacter sp. Leaf216 | WP_055908511.1 | + |
| 8 | Sphingobacterium sp. ML3W | WP_038699100.1 | + |
| 9 | Pedobacter sp. Leaf194 | A0A0Q5TVD2 | + |
| 10 | Sphingobacterium multivorum | WP_201664953.1 | + |
| 11 | Sphingobacterium sp. HMA12 | WP_104381007.1 | + |

| | | | |
|---|---|---|---|
| Note: In the table above, + represented a molar conversion rate greater than or less than 10%, and ++ represented a molar conversion rate greater than or equal to 10% and less than 20%. Among them, the molar conversion rate was the number of moles of the dipeptide in the system after the reaction/(the number of moles of the single amino acid + the number of moles of the dipeptide)×100%. | | | |

### Example 3

The 10 different Aet screened in Table 1 were subjected to analyze the sequence identity of the amino acid sequences, a highly conserved sequence YGISYPGFYST (SEQ ID NO: 1) was identified, which was structurally located near the active center and represented relatively important amino acids. Subsequent sequence analysis of the enzymes that showed no activity during screening revealed that the aforementioned conserved sequences were not found simultaneously in most of the inactive enzymes, thereby suggesting a potential correlation with the dipeptide synthesis function.

To further confirm that the highly conserved sequence from SEQ ID NO: 2 to SEQ ID NO: 11 was the key to catalyzing the synthesis of the Gly-Tyr dipeptide, we mutated the amino acids in this conserved region to amino acids of the same type (Table 2) and performed activity verification using the same reaction system as in Example 2.

**Table 2:**

| SEQ ID NO: | Conserved sequence | Mutated to | Conversion rate |
|---|---|---|---|
| 2 | YGISYPGFYST | A | 0 |
| | YGISYPGFYST | A | 0 |
| | YGISYPGFYST | S | 0 |
| 3 | YGISYPGFYST | A | 0 |
| | YGISYPGFYST | A | 0 |
| | YGISYPGFYST | S | 0 |
| 4 | YGISYPGFYST | A | 0 |
| | YGISYPGFYST | A | 0 |
| | YGISYPGFYST | S | 0 |
| 5 | YGISYPGFYST | A | 0 |
| | YGISYPGFYST | A | 0 |
| | YGISYPGFYST | S | 0 |
| 6 | YGISYPGFYST | A | 0 |
| | YGISYPGFYST | A | 0 |
| | YGISYPGFYST | S | 0 |
| 7 | YGISYPGFYST | A | 0 |
| | YGISYPGFYST | A | 0 |
| | YGISYPGFYST | S | 0 |
| 8 | YGISYPGFYST | A | 0 |
| | YGISYPGFYST | A | 0 |
| | YGISYPGFYST | S | 0 |
| 9 | YGISYPGFYST | A | 0 |
| | YGISYPGFYST | A | 0 |
| | YGISYPGFYST | S | 0 |
| 10 | YGISYPGFYST | A | 0 |
| | YGISYPGFYST | A | 0 |
| | YGISYPGFYST | S | 0 |
| 11 | YGISYPGFYST | A | 0 |
| | YGISYPGFYST | A | 0 |
| | YGISYPGFYST | S | 0 |

From the experimental results, it could be seen that when some amino acids in the conserved sequence were mutated to corresponding amino acids with similar spatial structures or chemical properties, the catalytic activity of the enzyme was substantially lost, indicating that these sequences were highly conserved, which was a basic feature of SEQ ID NO: 2 to SEQ ID NO: 11 and also the basic framework of the enzyme for synthesizing dipeptides, being crucial for the synthesis of dipeptides.

### Example 4

The activity of synthesizing Gly-Gln dipeptide for SEQ ID NO: 4 to SEQ ID NO: 13 was investigated. The reaction system was as follows (1 mL): 12.5 mg (100 mM) glycine methyl ester hydrochloride, 14.6 mg (100 mM) glutamine, 62.5 µL of crude enzyme solution, 0.1 M Tris-HCI (pH 8.0), and reacted at 20°C for 2 h. The reaction system was heated at 80°C for 10 min, centrifuged, and then subjected to HPLC analysis. The reaction results were summarized as follows (Table 3):

**Table 3:**

| SEQ ID NO: | Sequence source | NCBI sequence number | Conversion rate |
|---|---|---|---|
| 4 | Sphingobacterium sp. SRCM116780 | WP_234514695.1 | ++++ |
| 5 | Sphingobacterium paramultivorum | WP_182332756.1 | ++ |
| 6 | Sphingobacterium siyangense | WP_120334743.1 | ++ |
| 7 | Sphingobacterium sp. Ag1 | WP_046675788.1 | +++ |
| 8 | Pedobacter sp. | RZK77448.1 | + |
| 9 | Pedobacter sp. Leaf216 | WP_055908511.1 | ++ |
| 10 | Sphingobacterium sp. ML3W | WP_038699100.1 | + |
| 11 | Pedobacter sp. Leaf194 | A0A0Q5TVD2 | + |
| 12 | Sphingobacterium multivorum | WP_201664953.1 | + |
| 13 | Sphingobacterium sp. HMA12 | WP_104381007.1 | ++ |

| | | | |
|---|---|---|---|
| Note: In the table above, + represented a molar conversion rate of less than 20%, ++ represented a molar conversion rate of greater than or equal to 20% and less than 30%, +++ represented a molar conversion rate of greater than or equal to 30% and less than 40%, and ++++ represented a molar conversion rate of greater than 40%. | | | |

### Example 5

The activity of synthesizing Ala-Gln dipeptide for SEQ ID NO: 4 to SEQ ID NO: 13 was investigated. The reaction system was as follows (1 mL): 13.9 mg (100 mM) alanine methyl ester hydrochloride, 14.6 mg (100 mM) glutamine, 62.5 µL of crude enzyme solution, 0.1 M Tris-HCI (pH 8.0), and reacted at 20°C for 2 h. The reaction system was heated at 80°C for 10 min, centrifuged, and then subjected to HPLC analysis. The reaction results were summarized as follows (Table 4):

**Table 4:**

| SEQ ID NO: | Sequence source | NCBI sequence number | Conversion rate |
|---|---|---|---|
| 2 | Sphingobacterium sp. SRCM116780 | WP_234514695.1 | ++++ |
| 3 | Sphingobacterium paramultivorum | WP_182332756.1 | +++ |
| 4 | Sphingobacterium siyangense | WP_120334743.1 | ++ |
| 5 | Sphingobacterium sp. Ag1 | WP_046675788.1 | ++++ |
| 6 | Pedobacter sp. | RZK77448.1 | + |
| 7 | Pedobacter sp. Leaf216 | WP_055908511.1 | + |
| 8 | Sphingobacterium sp. ML3W | WP_038699100.1 | ++ |
| 9 | Pedobacter sp. Leaf194 | A0A0Q5TVD2 | ++ |
| 10 | Sphingobacterium multivorum | WP_201664953.1 | + |
| 11 | Sphingobacterium sp. HMA12 | WP_104381007.1 | ++ |

| | | | |
|---|---|---|---|
| Note: In the table above, + represented a molar conversion rate of less than 20%, ++ represented a molar conversion rate of greater than or equal to 20% and less than 30%, +++ represented a molar conversion rate of greater than or equal to 30% and less than 40%, and ++++ represented a molar conversion rate of greater than 40%. | | | |

### Example 6

Using SEQ ID NO: 2 and SEQ ID NO: 5 with high activity, the reactions for synthesizing Gly-Tyr, Gly-Gln, and Ala-Gln in the above embodiment were performed, wherein the substrate was changed from methyl ester hydrochloride to ethyl ester hydrochloride and isopropyl ester hydrochloride. The reaction system was as follows (1 mL): 100 mM ethyl ester hydrochloride/isopropyl ester hydrochloride, 100 mM glutamine/tyrosine, 62.5 µL of crude enzyme solution, 0.1 M Tris-HCI (pH 8.0), and reacted at 20°C for 2 h. The reaction system was heated at 80°C for 10 min, centrifuged, and then subjected to HPLC analysis. The reaction results were summarized as follows (Table 5):

**Table 5:**

| SEQ ID NO: | Synthesis reaction | Ester | Conversion rate |
|---|---|---|---|
| 2 | Gly-Tyr | Glycine ethyl ester hydrochloride | + |
| | | Glycine isopropyl ester hydrochloride | + |
| | Gly-Gln | Glycine ethyl ester hydrochloride | + |
| | | Glycine isopropyl ester hydrochloride | + |
| | Ala-Gin | Alanine ethyl ester hydrochloride | + |
| | | Alanine isopropyl ester hydrochloride | + |
| 5 | Gly-Tyr | Glycine ethyl ester hydrochloride | + |
| | | Glycine isopropyl ester hydrochloride | + |
| | Gly-Gln | Glycine ethyl ester hydrochloride | + |
| | | Glycine isopropyl ester hydrochloride | + |
| | Ala-Gin | Alanine ethyl ester hydrochloride | + |
| | | Alanine isopropyl ester hydrochloride | + |

| | | | |
|---|---|---|---|
| Note: In the table above, + represented a molar conversion rate greater than 5% and less than 20%. | | | |

In the above reaction, for the synthesis reactions of Gly-Tyr, Gly-Gln, and Ala-Gln, the substrate was changed from the methyl ester hydrochloride to the ethyl ester hydrochloride and the isopropyl ester hydrochloride, the corresponding activities were also clearly detected (although the enzyme activity decreased). It was proved that the methyl ester hydrochloride could be replaced with other derived esters.

### Example 7

The activity of synthesizing Gly-Gly dipeptide by SEQ ID NO: 2 to SEQ ID NO: 11 was investigated. The reaction system was as follows (1 mL): 100 mM glycine methyl ester hydrochloride, 100 mM glycine, 62.5 µL of crude enzyme solution, 0.1 M Tris-HCI (pH 8.0), and reacted at 20°C for 2 h. The reaction system was heated at 80°C for 10 min, centrifuged, and then subjected to HPLC analysis. The reaction results were summarized as follows (Table 6):

**Table 6:**

| SEQ ID NO: | Sequence source | NCBI sequence number | Conversion rate |
|---|---|---|---|
| 2 | Sphingobacterium sp. SRCM116780 | WP_234514695.1 | ++++ |
| 3 | Sphingobacterium paramultivorum | WP_182332756.1 | +++ |
| 4 | Sphingobacterium siyangense | WP_120334743.1 | +++ |
| 5 | Sphingobacterium sp. Ag1 | WP_046675788.1 | ++++ |
| 6 | Pedobacter sp. | RZK77448.1 | + |
| 7 | Pedobacter sp. Leaf216 | WP_055908511.1 | +++ |
| 8 | Sphingobacterium sp. ML3W | WP_038699100.1 | ++++ |
| 9 | Pedobacter sp. Leaf194 | A0A0Q5TVD2 | +++ |
| 10 | Sphingobacterium multivorum | WP_201664953.1 | +++ |
| 11 | Sphingobacterium sp. HMA12 | WP_104381007.1 | ++ |

| | | | |
|---|---|---|---|
| Note: In the table above, + represented a molar conversion rate of less than 20%, ++ represented a molar conversion rate of greater than or equal to 20% and less than 30%, +++ represented a molar conversion rate of greater than or equal to 30% and less than 40%, and ++++ represented a molar conversion rate of greater than 40%. | | | |

### Example 8

For SEQ ID NO: 2 and SEQ ID NO: 5 with good activity in the above embodiments, reactions were performed with 20 amino acid methyl ester hydrochlorides and 20 amino acids to investigate the substrate spectrum range. The reaction system was as follows (1 mL): 100 mM different amino acid methyl ester hydrochlorides (the first column in the table below), 14.6 mg (100 mM) different amino acids (the first row in the table below), 62.5 µL of crude enzyme solution, 0.1 M Tris-HCI (pH 8.0), and reacted at 20°C for 2 h. The reaction system was heated at 80°C for 10 min, centrifuged, and then subjected to LC-MS analysis. The reaction results were summarized as follows (Table 7 and Table 8):

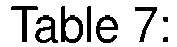

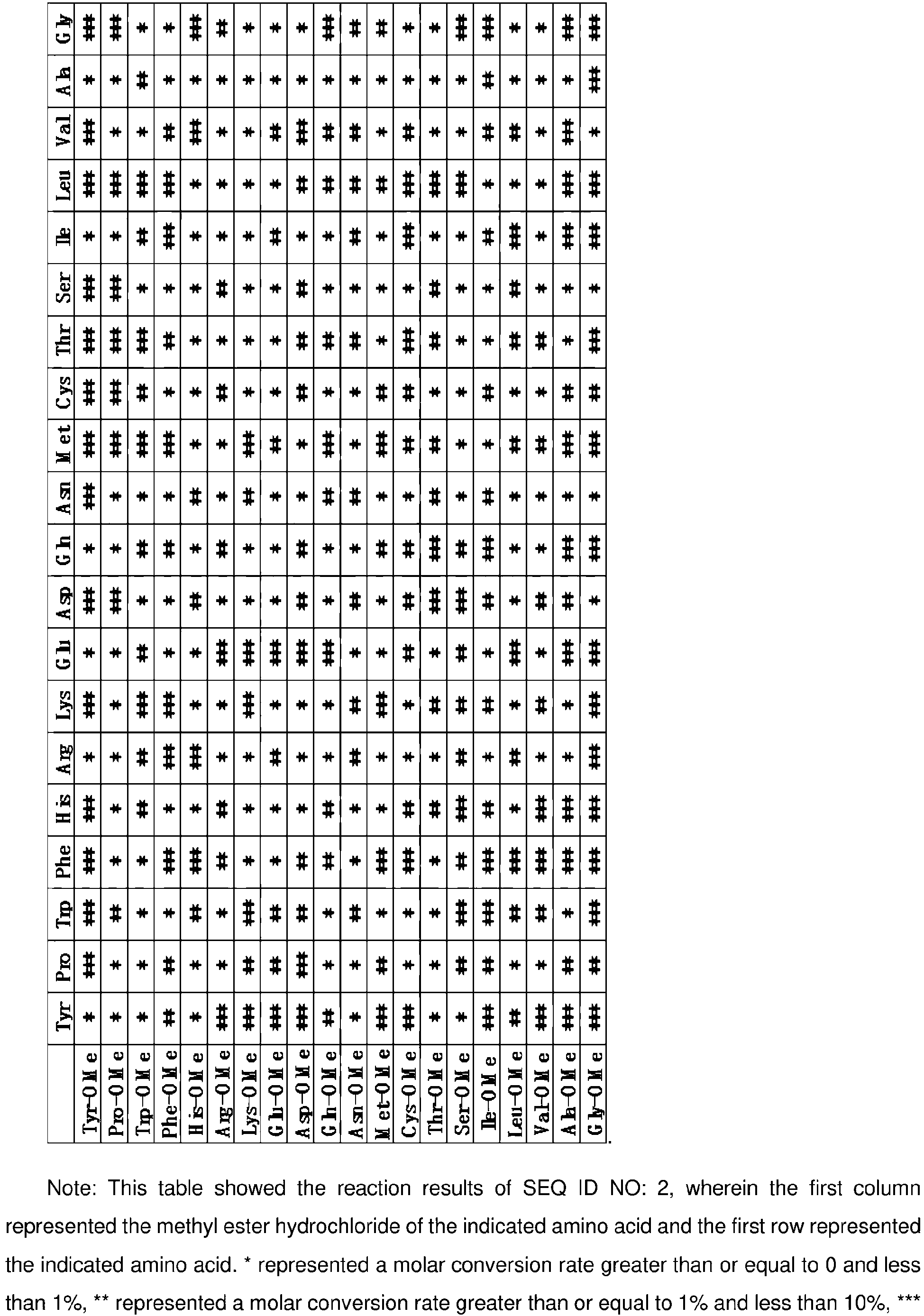

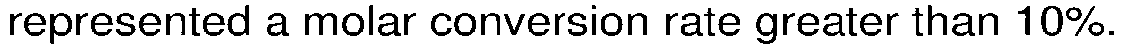

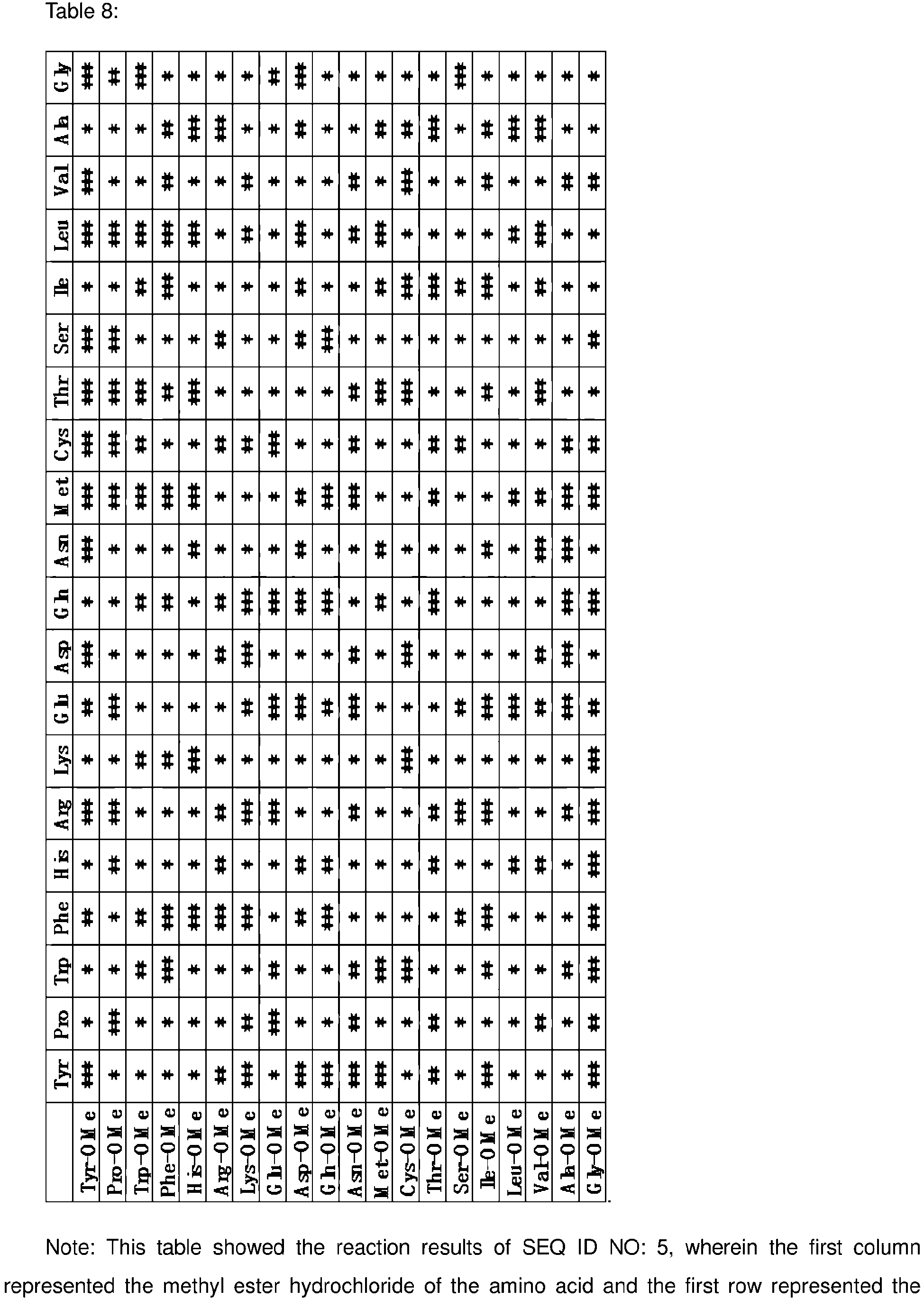

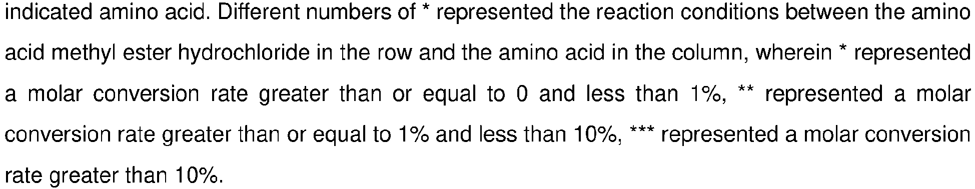

Among them, a molar conversion rate of the reaction >0 indicated that the reaction could synthesize dipeptides, and as shown in the above results, SEQ ID NO: 2 and SEQ ID NO: 5 had a broad substrate spectrum and could be used for the synthesis of a plurality of functional dipeptides. Additionally, the substrate spectrum verification as described above was also performed on the 9 enzymes from SEQ ID NO: 3 to SEQ ID NO: 4 and SEQ ID NO: 6 to SEQ ID NO: 11, respectively. The results showed that these 8 enzymes also exhibited dipeptide synthesis activity for most reactions, although the activity was generally low (1<molar conversion rate<10%).

### Example 9

The synthesis reaction amplification of Gly-Tyr, Gly-Gln, and Ala-Gln was performed on SEQ ID NO: 2. The reaction system was as follows (1 L): 400 mM glycine methyl ester hydrochloride and alanine methyl ester hydrochloride, 400 mM tyrosine and glutamine, 30 mL of crude enzyme solution, 0.1 M Tris-HCI (pH 8.0), and reacted at 20°C for 2 h. After the reaction was completed, post-treatment separation and purification of the reaction system were performed, and the obtained final product was sent for HPLC and NMR detection of purity and content, wherein the results were summarized as follows (Table 9):

**Table 9:**

| SEQ ID NO: | Synthetic product | Molar conversion rate | Product purity | Separation yield |
|---|---|---|---|---|
| 2 | Gly-Tyr | >70% | >99.8 | >60% |
| | Gly-Gln | >70% | >99.8 | >60% |
| | Ala-Gin | >70% | >99.8 | >60% |

From the above description, it could be seen that the above embodiments of the present invention achieved the following technical effects: (1) in the present invention, 10 enzymes with dipeptide synthesis activity, namely SEQ ID NO: 2 to SEQ ID NO: 11, obtained through enzyme screening comprised highly conserved amino acid sequence regions that were related to dipeptide synthesis activity and constituted the basic framework of the catalytic activity of these 10 enzymes.

(2) SEQ ID NO: 2 to SEQ ID NO: 11 could be used not only for synthesizing Gly-Tyr but also for synthesizing a plurality of functional dipeptides including but not limited to Gly-Gln, Gly-Gly, and Ala-Gln, the substrate spectrum was broad, and some enzymes exhibited high synthesis efficiency.

(3) The enzyme obtained in the present invention could be well used for industrial scale-up, with low cost and high yield, achieving true green chemistry.

The embodiments described above are merely preferred embodiments of the present invention and are not intended to limit the present invention. Various modifications and variations of the present invention can be made by those skilled in the art. Any modification, equivalent replacement, improvement, and the like made within the spirit and principle of the present invention shall be included in the scope of protection of the present invention.

## Claims

1. A method for preparing a dipeptide, wherein the method comprises:
using an α-amino acid ester acyltransferase to perform a dipeptide synthesis reaction, and obtaining the dipeptide;
wherein the α-amino acid ester acyltransferase has a conserved region with the amino acid sequence as shown in SEQ ID NO: 1.

2. The method according to claim 1, wherein the α-amino acid ester acyltransferase is selected from any one of the α-amino acid ester acyltransferases with amino acid sequences as shown in SEQ ID NOs: 2-11.

3. The method according to claim 1, wherein substrates for the dipeptide synthesis reaction comprise a substrate as an acyl donor and a substrate as a nucleophile, wherein the substrate as an acyl donor is selected from an amino acid ester hydrochloride, and the substrate as a nucleophile is an amino acid.

4. The method according to claim 3, wherein, the amino acid ester hydrochloride comprises an amino acid methyl ester hydrochloride, an amino acid ethyl ester hydrochloride, or an amino acid isopropyl ester hydrochloride.

5. The method according to claim 3, wherein, the amino acid in the amino acid ester hydrochloride and the amino acid as a nucleophile are each independently selected from glycine, alanine, valine, leucine, isoleucine, serine, threonine, cysteine, methionine, asparagine, glutamine, aspartic acid, glutamic acid, lysine, arginine, histidine, phenylalanine, tryptophan, proline, or tyrosine.

6. The method according to claim 1, wherein the dipeptide comprises Gly-Gln, Gly-Tyr, Gly-Gly, Ala-Gin or Arg-Trp.

7. The method according to any one of claims 1 to 6, wherein the dipeptide synthesis reaction is performed in an alkaline environment.

8. The method according to any one of claims 1 to 6, wherein a reaction temperature for the dipeptide synthesis reaction is 20-25°C; a reaction time is 1.5-2h.

9. The method according to any one of claims 1 to 6, wherein after the dipeptide synthesis reaction, the method further comprises heating and deactivating the α-amino acid ester acyltransferase,
a temperature for the heating and deactivating is 75-85°C; and
a time for the heating and deactivating is 10-15 min.

10. The method according to any one of claims 1 to 6, wherein based on the mass ratio of wet bacterial sludge to the substrate, the mass ratio of the wet bacterial sludge comprising the α-amino acid ester acyltransferase to the substrate for the dipeptide synthesis reaction is 1:1-10.

11. A method for preparing an α-amino acid ester acyltransferase, wherein cloning the gene of the α-amino acid ester acyltransferase in the method of claim 1 into an expression vector, and obtaining a recombinant vector;
transforming the recombinant vector into a host cell, and obtaining a recombinant strain;
culturing and inducing the recombinant strain to express the α-amino acid ester acyltransferase, and obtaining a culture solution;
subjecting the culture solution to ultrasonic disruption and centrifugation, and obtaining the α-amino acid ester acyltransferase.

12. The method according to claim 11, the 3' end or 5' end of the gene of the α-amino acid ester acyltransferase in the recombinant vector comprises a tag gene.

13. The method according to claim 12, the tag gene comprises a DNA sequence encoding MBP protein, NusA protein, Trx protein, SUMO protein, DsbA protein, TF protein or GST protein.
